# EUROPÄISCHE PATENTSCHRIFT

(11) **EP 2 756 854 B1**
(45) Veröffentlichungstag und Bekanntmachung des Hinweises auf die Patenterteilung: **08.11.2017**
(21) Anmeldenummer: 14151284.8
(22) Anmeldetag: 15.01.2014
(51) Int. Cl.: A61M 1/16, A61J 1/10, A61M 39/26

(54) **Medizinischer Fluidbeutel**
Medical fluid bag
Poche de fluide médicale

(30) Priorität: 17.01.2013 DE 102013100479
(43) Veröffentlichungstag der Anmeldung: 23.07.2014
(73) Patentinhaber: B. Braun Avitum AG, 34212 Melsungen (DE)
(72) Erfinder: Wesseler, Matthias, 49326 Melle (DE)
(74) Vertreter: Winter, Brandl, Fürniss, Hübner, Röss, Kaiser, Polte - Partnerschaft mbB

(56) Entgegenhaltungen:
- EP-A1- 1 125 597
- EP-A2- 0 928 621
- US-A- 5 509 433

## Beschreibung

Die vorliegende Erfindung betrifft medizinische Fluidbeutel gemäß Anspruch 1.

Neben Glasflaschen und Kunststoffflaschen auf Polyethylenbasis werden in der modernen Infusionstherapie auch Infusionsbeutel aus Kunststoff typischerweise auf Basis von PE und PP, sogenannte Fluidbeutel, für die parenterale Zufuhr von für eine Infusionstherapie erforderliche Stoffe im klinischen Alltag eingesetzt.

Für die Verwendung von Fluidbeuteln anstelle von Glas- oder Kunststoffflaschen spricht eine Reihe von Vorteilen:
a) Neben ihrem geringen Gewicht und niedrigen Herstellungskosten sind Fluidbeutel anwenderfreundlich in dem Sinne, dass ein Leerlaufen des Infusionssystems, insbesondere von deren Tropfkammer, nicht möglich ist, da die Fluidbeutel kollabieren und am Infusionsende ein Stopp der Flüssikeitssäule entsteht. Somit existiert ein geschlossenes System und eine Luftembolie ist praktisch nicht möglich.
b) Beim Einsatz von Fluidbeuteln ist eine keimträchtige Belüftung der Infusionsbeutel nicht erforderlich.
c) Falls zusätzliche Medikamente in die Infusion gegeben werden sollen, ist der Inhalt leicht zu durchmischen.
d) Darüber hinaus sind Fluidbeutel flexibel, so dass sie einfach für Druckinfusionen verwendet werden können, falls schnell Bedarf besteht, Volumen zu substituieren.
e) Fluidbeutel sind ferner transparent, wodurch eventuelle Verfärbungen oder Ausfällungen erkennbar sind, was insbesondere bei falscher Lagerung oder Überlagerung von Infusionslösungen passieren kann.

Obwohl früher Fluidbeutel für Infusionszwecke auch aus PVC hergestellt wurden, werden mittlerweile Infusionsbeutel aus einem Folienverbund, hergestellt aus Polyethylen und Polypropylen, verwendet.

Derartige Fluidbeutel lassen sich prinzipiell zusammen mit ihrem Inhalt - z. B. physiologische Kochsalzlösung - problemlos heißdampfsterilisieren.

Die EP 1 125 597 A1 beschreibt ein dampfsterilisierbares Flüssigkeitsventil für die Flüssigkeitsentnahme aus einem Behälter, der sterile Flüssigkeiten enthalten kann. Das Ventil weist ein Kupplungselement auf, das eine Kupplungshülse zum Ankuppeln eines Konnektors aufweist, mit einer flexiblen Ventilscheibe, die durch einen zentralen Dorn abgestützt ist und mit ihrem Rand gegen einen Ventilsitz drückt und einer die Kupplungshülse überdeckenden entfernbaren Kappe, wobei eine die Ventilscheibe auf dem Dorn zentriert haltende Haltevorrichtung vorgesehen ist.

Grundsätzlich müssen Behältnisse für medizinische Flüssigkeiten nach Abfüllung der Flüssigkeiten sterildicht verschlossen sein. Dies geschieht beispielsweise durch ein Ventil o. Ä., welches den Auslaufstutzen des Behältnisses gegen Auslaufen verschließt. Dieses Ventil wiederum muss ebenso sterildicht vor äußeren Einflüssen geschützt sein. Hierfür werden fabrikseitig kappenförmige Originalitätsverschlüsse stoffschlüssig aufgebracht. Im Inneren dieser Kappen befindet sich jedoch ein Hohlraum, der bei einer Heißdampfsterilisation nicht sicher mit Flüssigkeit benetzt wird, was jedoch die Grundvoraussetzung für eine Heißdampfsterilisation ist.

Im Stand der Technik wurden deshalb in derartige Originalitätsverschlüsse zum Zwecke der Heißdampfsterilisation Flüssigkeitstropfen gezielt eingebracht, was jedoch einen deutlichen Mehraufwand bei der Serienabpackung von Infusionsflüssigkeiten im Fluidbeutel bedeutet.

Darüber hinaus wurde alternativ die Gammastrahlensterilisation eingesetzt, welche jedoch für den Hersteller bereits apparativ einen bedeutenden Aufwand darstellt, neben hohen Kosten und Strahlenschutzauflagen, die von Gesetzes wegen zu erfüllen sind.

Vor diesem Hintergrund ist es die Aufgabe der vorliegenden Erfindung, medizinische Fluidbeutel zur Verfügung zu stellen, welche durch eine einfache Heißdampfsterilisation vollständig, also inklusive sämtlicher Hohlräume in den Originalitätsverschlüssen heißdampfsterilisiert werden können.

Die Lösung dieser Aufgabe erfolgt durch die kennzeichnenden Merkmale des Anspruchs 1.

Insbesondere betrifft die vorliegende Erfindung einen medizinischen Fluidbeutel, welcher wenigstens einen Auslaufstutzen aufweist, der mit einer schaltbaren Ventileinrichtung zur Außenumgebung hin verschlossen ist, die mittels einer sterildicht auf dem Auslaufstutzen angeordneten Verschlusseinrichtung abgekapselt ist, wobei die Verschlusseinrichtung ein auf dem Auslaufstutzen angeordnetes Basisteil und eine Abbrechkappe aufweist, wobei Basisteil und Abbrechkappe über eine als Sollbruchstelle/-linie dienende Verbindung einstückig miteinander verbunden sind, wobei die Ventileinrichtung in ihrem Inneren einen Ventilkörper (Ventilstößel) aufweist, der die Ventileinrichtung nach Entfernen der Abbrechkappe bei bestimmungsgemäßem Gebrauch des Fluidbeutels auf Druck öffnet, wobei im Inneren der Abbrechkappe der Verschlusseinrichtung ein Ventilbetätigungselement vorgesehen ist, welches nach Verbindung von Auslaufstutzen mit Verschlusseinrichtung den Ventilkörper (Ventilstößel) zwangsbetätigt und so die Ventilreinrichtung derart öffnet, dass eine fluidische Verbindung zwischen Fluidbeutel und einem von der Abbrechkappe abgekapselten Hohlraum insbesondere eines Konnektionsbereiches zum Anschluss eines Anschlusssystems - beispielsweise ein Infusionsbesteck - an den Fluidbeutel vorliegt.

Eine alternative Lösung der obigen Aufgabe ist durch einen medizinischen Fluidbeutel gemäß Anspruch 9 gegeben. Insbesondere besteht diese unabhängige Lösung der obigen Aufgabe in einem medizinischen Fluidbeutel, welcher wenigstens einen Auslaufstutzen aufweist, der mit einer schaltbaren Ventileinrichtung zur Außenumgebung hin verschlossen ist, die mittels einer sterildicht auf dem Auslaufstutzen angeordneten Verschlusseinrichtung abgekapselt ist, wobei die Verschlusseinrichtung ein auf dem Auslaufstutzen angeordnetes Basisteil und eine Abbrechkappe aufweist, wobei Basisteil und Abbrechkappe über eine als Sollbruchstelle/-linie dienende Verbindung einstückig miteinander verbunden sind, wobei die Ventileinrichtung nach Entfernen der Abbrechkappe bei bestimmungsgemäßem Gebrauch des Fluidbeutels mittels eines Anschlusssystems öffnet, wobei im Inneren der Abbrechkappe der Verschlusseinrichtung ein Ventilbetätigungselement vorgesehen ist, welches nach Verbindung von Auslaufstutzen mit Verschlusseinrichtung die Ventilreinrichtung derart zwangsöffnet, dass eine fluidische Verbindung zwischen Fluidbeutel und einem von der Abbrechkappe abgekapselten Hohlraum inbesondere eines Konnektionsbereiches zum Anschluss des Anschlusssystems - beispielsweise ein Infusionsbesteck - an den Fluidbeutel vorliegt.

Durch die obigen Maßnahmen wird sichergestellt, dass eine Flüssigkeitsbenetzung sämtlicher relevanter Hohlräume der Verschlüsse, insbesondere der Originalitätsverschlüsse der Fluidbeutel, erfolgt, wodurch eine vollständige und zuverlässige Heißdampfsterilisation der vorliegenden Fluidbeutel ermöglicht wird.

Mit den erfindungsgemäßen Fluidbeuteln sind weder zusätzliche Arbeitsgänge noch zusätzliche Kontrollen oder hohe Investitionskosten für alternative Sterilisierungsmaßnahmen erforderlich.

Die erfindungsgemäßen Fluidbeutel weisen somit während des Sterilisationsprozesses ein geöffnetes Ventil zur sicheren Sterilisation des Ventilinnenraumes und des Kappeninnenraums in Verbindung mit einer sofortigen Dichtheit des Ventils nach dem Entfernen des Originalitätsverschlusses auf.

Hierdurch wird ein preisgünstiges Fertigungsverfahren, nämlich eine gleichzeitige Vorbereitung für den Sterilisationsprozess sowie eine sichere Heißdampfsterilisation des Innenraumes des Originalitätsverschlusses gewährleistet. Dies geschieht im Wesentlichen dadurch, dass die erfindungsgemäßen Fluidbeutel, welche für medizinische und sonstige Anwendungen geeignet sind, durch ein schaltbares Ventil und eine dieses abdeckende, z. B. topfförmigen Originalitätsverschluss verschlossen ist, bei dem ein in der Abbrechkappe des Originalitätsverschlusses enthaltener Betätigungsstift das Ventil während der Sterilisation offen hält, und so den Hohlraum im Originalitätsverschluss mit Flüssigkeit zur sicheren Heißdampfsterilisation versorgt.

Die Unteransprüche stellen bevorzugte Ausführungsformen der erfindungsgemäßen Fluidbeutel dar.

Weitere Vorteile und Merkmale der vorliegenden Erfindung ergeben sich aufgrund der Beschreibung eines Ausführungsbeispiels sowie anhand der Zeichnung.

Es zeigt:
- FIG. 1: einen Konnektionsbereich eines erfindungsgemäßen Fluidbeutels in einer Längsschnittansicht;
- FIG. 2: einen Konnektionsbereich mit entfernter Abbrechkappe und geschlossenem Ventil in Längsschnittansicht;
- FIG. 3: die Abbrechkappe gemäß FIG. 1 und 2 in perspektivischer Ansicht;
- FIG. 4: einen Konnektionsbereich eines erfindungsgemäßen Fluidbeutels mit einem elastischen Schlitzventil mit ungeöffnetem Originalitätsverschluss mit geöffnetem Ventil in Längsschnittansicht; und
- FIG. 5: eine Längsschnittansicht des Konnektionsbereiches eines erfindungsgemäßen Fluidbeutels gemäß FIG. 4 mit entfernter Abbrechkappe und geschlossenem Ventil in Längsschnittansicht.

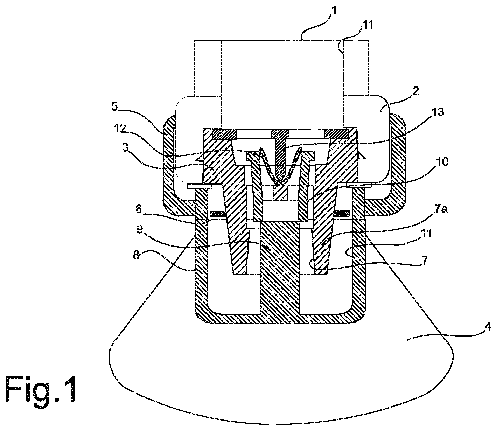

In FIG. 1 ist eine Längsschnittansicht eines Konnektionsbereiches 7a eines erfindungsgemäßen Fluidbeutels dargestellt. Das Gesamtsystem umfasst einen mit Flüssigkeit 11 gefüllten Fluidbeutel 1, dessen Auslaufstutzen 2 mit einem schaltbaren Ventil als Ventileinrichtung 3 verschlossen ist. Das schaltbare Ventil 3 ist zur Umgebung hin durch einen sterildicht auf dem Auslaufstutzen 2 befestigten Originalitätsverschluss als Verschlusseinrichtung 4 verschlossen.

Der Kunststofforiginalitätsverschluss 4 umfasst im Beispielsfalle ein glockenförmiges Basisteil 5 zur stoffschlüssigen Befestigung auf dem Auslaufstutzen 2 sowie eine im Beispielsfalle topfförmige Abbrechkappe 8, wobei beide Teile durch eine dünne, als Sollbruchstelle/-linie dienende Verbindung 6 einstückig miteinander verbunden sind.

Das schaltbare Ventil 3 verfügt über einen innenliegenden Ventilkörper/Ventilstößel 10, der, sobald er beispielsweise durch einen Luer-Konus in Richtung hin zum Beutel eingedrückt wird, das schaltbare Ventil (3) öffnet, um so den Fluidbeutel seinem bestimmungsgemäßen Gebrauch nach Anschluss eines Infusionsbestecks zuzuführen.

Um ein Öffnen des schaltbaren Ventils 3 und damit eine Flüssigkeitsverbindung zwischen dem Inhalt des Fluidbeutels 1 und einem Hohlraum 7 des Originalitätsverschlusses 4 sicherzustellen, muss das Ventil während des Heißdampfsterilisationsverfahrens geöffnet sein.

Dieses wird gemäß der vorliegenden Erfindung dadurch erreicht, dass im Inneren der Abbrechkappe 8 des Originalitätsverschlusses 4 ein Ventilbetätigungselement/-stift 9 angeordnet ist, der nach dem Prozess der stoffschlüssigen Verbindung, z. B. Reibschweißen, zwischen Auslaufstutzen 2 und Originalitätsverschluss 4 den innenliegenden Ventilstößel 10 des Ventils 3 eindrückt und somit das schaltbare Ventil 3 zwangsöffnet.

Durch das Öffnen des Ventils 3 ergibt sich eine fluidische Verbindung zwischen dem Fluidbeutel 1 und dem Hohlraum 7 im Originalitätsverschluss 4, wodurch Flüssigkeit 11 aus dem Fluidbeutel 1 in den Hohlraum 7 eintritt.

Durch diese Benetzung kann eine ordnungsgemäße und sichere Sterilisation mittels Heißdampfverfahren auch innerhalb des Originalitätsverschlusses 4 erfolgen.

Beim Öffnen des Originalitätsverschlusses 4 durch Abbrechen oder Abdrehen der Abbrechkappe 8 des Originalitätsverschlusses 4 wird der Ventilbetätigungsstift 9 außer Eingriff mit dem Ventilstößel 10 gebracht und das Ventil 3 schließt sofort.

Der in der Abbrechkappe 8 des Originalitätsverschlusses 4 verbleibende Flüssigkeitstropfen ist nicht störend, da darüber hinaus kein weiteres Fließen oder Nachtropfen der Infusionslösung aus dem Fluidbeutel 1 erfolgt.

Nach dem Entfernen der Abbrechkappe 8 des Originalitätsverschlusses 4 kann der Fluidbeutel 1 mit einem dem Fachmann wohlbekannten Anschlusssystem für die vorgesehene Verwendung, z. B. mit einem Infusionsbesteck, verbunden werden und einem Patienten eine Infusion angelegt werden.

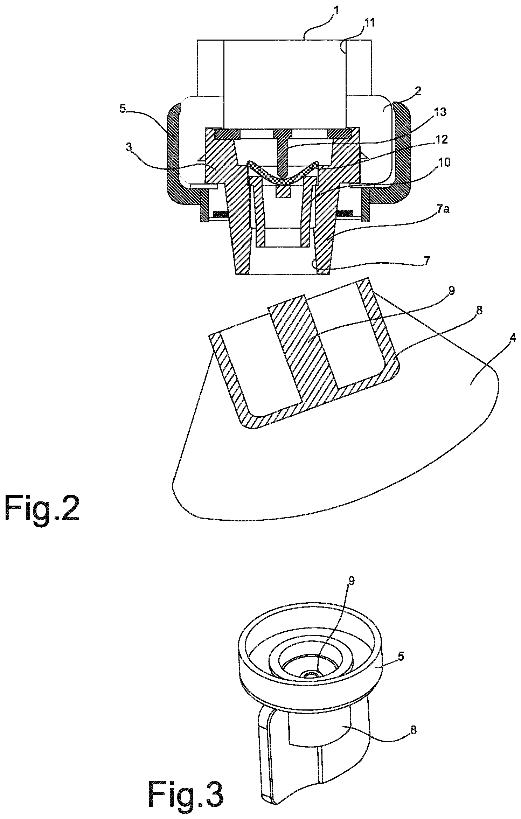

In den Figuren 2 und 3 ist der Zustand des Ventils 3 im geschlossenen Zustand nach dem Entfernen der Abbrechkappe 8 bzw. des Originalitätsverschlusses 4 gezeigt.

Im Beispielsfalle der Figuren 1 und 2 wird innerhalb der Ventileinrichtung 3 zusätzlich eine Membran als Schließelement 12 verwendet, die von einem fluidbeutelinneren Dorn 13 und dem Ventilstößel 10 in Position gehalten wird, um so über die Membran 12 den Ventilstößel 10 längs zu führen.

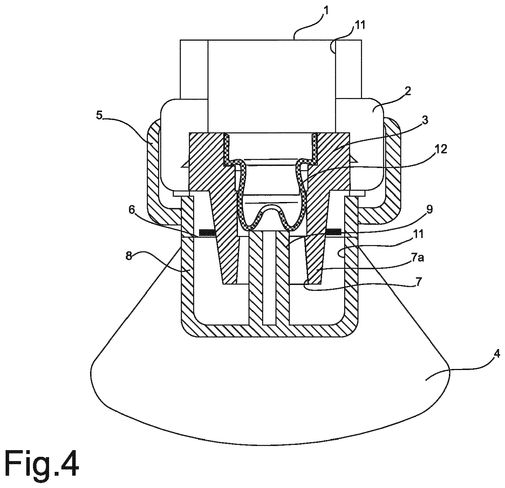

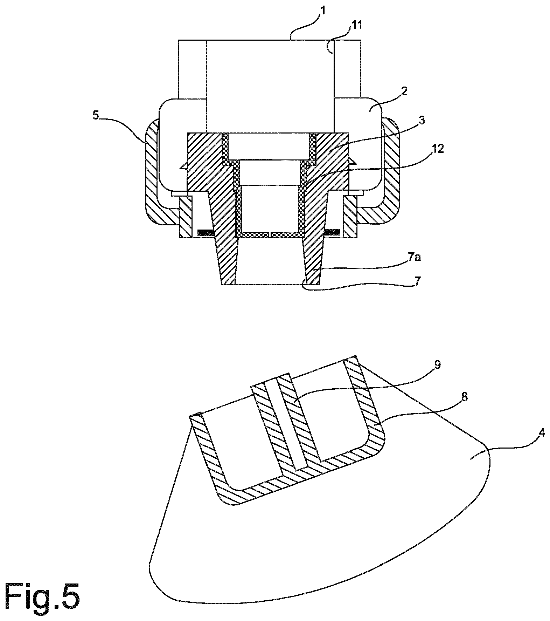

Alternativ kann die vorliegende Erfindung jedoch auch für einen Konnektionsbereich 7a mit elastischen Schlitzventilen als Ventileinrichtung 3 eingesetzt werden, was in den Figuren 4 und 5 gezeigt ist. D. h. in diesem Fall ist der Ventilkörper nicht als starrer Ventilstößel sondern als flexibles/elastisches Element ausgebildet, das sich bei Druckbelastung mittels des kappenseitigen Betätigungselements 9 deformiert und so die Ventilöffnung frei gibt.

Als Möglichkeiten für das Ventilbetätigungselement 9 kommen in allen Fällen zylindrisch massive, rohrförmige und profilförmige, insbesondere kreuzförmige und dreieckförmige sowie Y-förmige Querschnitte als Möglichkeit in Betracht.

Mit den erfindungsgemäßen Fluidbeuteln ist es somit möglich, auf einfache Weise eine vollständige Heißdampfsterilisierung eines Totraumes innerhalb eines Originalitätsverschlusses ohne zusätzliche Maßnahmen, wie gezieltes Einbringen von Flüssigkeiten vor dem Sterilisationsprozess, zu ermöglichen.

### Bezugszeichenliste

- 1: Fluidbeutel
- 2: Auslaufstutzen
- 3: Schaltbares Ventil/Ventileinrichtung
- 4: Originalitätsverschluss/Verschlusseinrichtung
- 5: Basisteil
- 6: Sollbruchstellen-Verbindung
- 7: Hohlraum
- 7a: Konnektionsbereich
- 8: Abbrechkappe
- 9: Ventilbetätigungselement
- 10: Ventilkörper/Ventilstößel
- 11: Flüssigkeit
- 12: Schließelement
- 13: Dorn

## Patentansprüche

1. Medizinischer Fluidbeutel, insbesondere Dialysefluidbeutel welcher wenigstens einen Auslaufstutzen (2) aufweist, der mit einer schaltbaren Ventileinrichtung (3) zur Außenumgebung hin verschlossen ist und mittels einer sterildicht auf dem Auslaufstutzen (2) angeordneten Verschlusseinrichtung (4) abgekapselt ist, die ein auf dem Auslaufstutzen (2) angeordnetes Basisteil (5) und eine Abbrechkappe (8) aufweist, wobei Basisteil (5) und Abbrechkappe (8) über eine als Sollbruchstelle dienende Verbindung (6) einstückig miteinander verbunden sind und
wobei die Ventileinrichtung (3) in ihrem Inneren einen Ventilkörper (10) aufweist, der die Ventileinrichtung (3) nach Entfernen der Abbrechkappe (8) bei bestimmungsgemäßem Gebrauch des Fluidbeutels (1) auf Druck öffnet, **dadurch gekennzeichnet, dass** im Inneren der Abbrechkappe (8) der Verschlusseinrichtung (4) ein Ventilbetätigungselement (9) vorgesehen ist, welches nach Verbindung von Auslaufstutzen (2) mit Verschlusseinrichtung (4) den Ventilkörper (10) zwangsbetätigt und so die Ventilreinrichtung (3) derart öffnet, dass eine fluidische Verbindung zwischen Fluidbeutel (1) und einem von der Abbrechkappe (8) abgekapselten Hohlraum (7) vorliegt.

2. Fluidbeutel nach Anspruch 1, **dadurch gekennzeichnet, dass** das Basisteil (5) der Verschlusseinrichtung (4) glockenförmig ausgebildet ist.

3. Fluidbeutel nach einem der vorhergehenden Ansprüche, **dadurch gekennzeichnet, dass** das Basisteil (5) stoffschlüssig auf dem Auslaufstutzen (2) angeordnet ist.

4. Fluidbeutel nach einem der vorhergehenden Ansprüche, **dadurch gekennzeichnet, dass** die Abbrechkappe (8) topfförmig ausgebildet ist und das Ventilbetätigungselement (9) als stoffeinstückig mit der Abbrechkappe (8) ausgebildeter stiftförmiger Vorsprung im abgekapselten Hohlraum vorgesehen ist, der in Montagezustand zwischen sich und einer Ventilöffnung einen Ausströmspalt in den abgekapselten Hohlraum freihält.

5. Fluidbeutel nach einem der vorhergehenden Ansprüche, **dadurch gekennzeichnet, dass** die Verschlusseinrichtung (4) als Originalitätsverschluss ausgebildet ist.

6. Fluidbeutel nach einem der vorhergehenden Ansprüche, **dadurch gekennzeichnet, dass** der Ventilkörper (10) ein hülsenförmiger Ventilstößel ist, dessen eine zum Fluidbeutel (1) hinweisende Stirnseite durch eine Membran verschlossen ist, welche durch einen zentralen Dorn (13) an der Beutelinnenseite abgestützt ist, um eine Axialbewegung des Ventilkörpers für ein Öffnen und Schließen des Ventils (3) zu führen.

7. Fluidbeutel nach einem der vorhergehenden Ansprüche, **dadurch gekennzeichnet, dass** er einschließlich des abgekapselten Raums heißdampfsterilisierbar ist.

8. Fluidbeutel nach einem der vorhergehenden Ansprüche, **dadurch gekennzeichnet, dass** sämtliche Bauteile des Fluidbeutels (1), des Ventils (3) und der Verschlusseinrichung (4) aus thermoplastischen Kunststoffen gebildet sind.

9. Medizinischer Fluidbeutel, insbesondere Dialysefluidbeutel, welcher wenigstens einen Auslaufstutzen (2) aufweist, der mit einer schaltbaren Ventileinrichtung (3) zur Außenumgebung hin verschlossen ist und mittels einer sterildicht auf dem Auslaufstutzen (2) angeordneten Verschlusseinrichtung (4) abgekapselt ist, die ein auf dem Auslaufstutzen (2) angeordnetes Basisteil (5) und eine Abbrechkappe (8) aufweist, wobei Basisteil (5) und Abbrechkappe (8) über eine als Sollbruchstelle dienende Verbindung (6) einstückig miteinander verbunden sind und
wobei die Ventileinrichtung (3) nach Entfernen der Abbrechkappe (8) bei bestimmungsgemäßem Gebrauch des Fluidbeutels (1) mittels eines Anschlusssystems öffnet, **dadurch gekennzeichnet, dass** im Inneren der Abbrechkappe (8) der Verschlusseinrichtung (4) ein Ventilbetätigungselement (9) vorgesehen ist, welches nach Verbindung von Auslaufstutzen (2) mit Verschlusseinrichtung (4) die Ventilreinrichtung (3) derart (zumindest temporär) öffnet, dass eine fluidische Verbindung zwischen Fluidbeutel (1) und einem Hohlraum (7) eines Konnektionsbereiches (7a) zum Anschluss eines Anschlusssystems an den Fluidbeutel (1) vorliegt.

10. Fluidbeutel nach einem der vorstehenden Ansprüche, **dadurch gekennzeichnet, dass** das Ventilbetätigungselement (9) ein axial vorragender Stift ist, der einen zylindrisch massiven, rohrförmigen, profilförmigen, insbesondere kreuzförmigen, dreieckförmigen oder Y-förmigen Querschnitt aufweist.

## Claims

1. Medical fluid bag, in particular dialysis fluid bag comprising at least one outlet nozzle (2) which is closed off from the external surroundings via a switchable valve unit (3) and which is encapsulated by a locking unit (4) which is arranged on the outlet nozzle (2) in a sterile-sealed manner, wherein the locking unit (4) comprises a base part (5) arranged on the outlet nozzle (2) and a break-off cap (8), wherein the base part (5) and the break-off cap (8) are integrally connected via a connection (6) serving as predetermined breaking point, and
wherein the valve unit (3) comprises on its inside a valve body (10) which opens the valve unit (3) when pressure is applied after removing the break-off cap (8) for intended use of the fluid bag (1), **characterized in that** a valve actuating element (9) is provided on the inside of the break-off cap (8) of the locking unit (4), wherein the valve actuating element (9) forcibly actuates the valve body (10) after connection of the outlet nozzle (2) with the locking unit (4) and thus opens the valve unit (3) such that a fluidic connection is present between the fluid bag (1) and a cavity (7) encapsulated from the break-off cap (8).

2. Fluid bag according to claim 1, **characterized in that** the base part (5) of the locking unit (4) is bell shaped.

3. Fluid bag according to one of the preceding claims, **characterized in that** the base part (5) is arranged in a firmly bonded manner on the outlet nozzle (2).

4. Fluid bag according to one of the preceding claims, **characterized in that** the break-off cap (8) is cup shaped and the valve actuating element (9) is provided as a pin-shaped projection integrally formed with the break-off cap (8) in the encapsulated cavity, wherein in the mounting state, this projection keeps clear a flow-out gap in the encapsulated cavity between itself and a valve opening.

5. Fluid bag according to one of the preceding claims, **characterized in that** the locking unit (4) is formed as tamper-evident closure.

6. Fluid bag according to one of the preceding claims, **characterized in that** the valve body (10) is a sleeve-shaped valve plunger whose one front side facing the fluid bag (1) is closed off by a membrane which is supported by a central bolt (13) at the inner bag side to guide an axial movement of the valve body for opening and closing of the valve (3).

7. Fluid bag according to one of the preceding claims, **characterized in that** including the encapsulated space, it can be sterilized with superheated steam.

8. Fluid bag according to one of the preceding claims, **characterized in that** all components of the fluid bag (1), of the valve (3) and of the locking unit (4) are made of thermoplastic plastics.

9. Medical fluid bag, in particular dialysis fluid bag comprising at least one outlet nozzle (2) which is closed off from the external surroundings via a switchable valve unit (3) and which is encapsulated by a locking unit (4) which is arranged on the outlet nozzle (2) in a sterile-sealed manner, wherein the locking unit (4) comprises a base part (5) arranged on the outlet nozzle (2) and a break-off cap (8), wherein the base part (5) and the break-off cap (8) are integrally connected via a connection (6) serving as predetermined breaking point, and
wherein the valve unit (3) opens via a connection system after removing the break-off cap (8) for intended use of the fluid bag (1), **characterized in that** a valve actuating element (9) is provided on the inside of the break-off cap (8) of the locking unit (4), wherein the valve actuating element (9) opens (at least temporarily) the valve unit (3) after connection of the outlet nozzle (2) with the locking unit (4) such that a fluidic connection is present between the fluid bag (1) and a cavity (7) of a connection region (7a) for connecting a connection system to the fluid bag (1).

10. Fluid bag according to one of the preceding claims, **characterized in that** the valve actuating element (9) is an axially projecting pin which has a cylindrically massive, tube-shaped, profile-shaped, in particular cross-shaped, triangularly-shaped, or Y-shaped cross section.

## Revendications

1. Poche de fluide médicale, en particulier poche de fluide de dialyse laquelle présente au moins une tubulure de sortie (2), qui est fermée vers l'environnement extérieur avec un dispositif de soupape commutable (3) et est encapsulée au moyen d'un dispositif de fermeture (4) disposé de manière stérile et étanche sur la tubulure de sortie (2), qui présente un élément de base (5) agencé sur la tubulure de sortie (2) et un capuchon cassable (8),
dans laquelle l'élément de base (5) et le capuchon cassable (8) sont reliés l'un à l'autre d'un seul tenant par le biais d'une liaison (6) servant de section de rupture intentionnelle, et
dans laquelle le dispositif de soupape (3) présente en son intérieur un corps de soupape (10) qui ouvre sur pression le dispositif de soupape (3) après le retrait du capuchon cassable (8) en cas d'utilisation conforme à l'affectation de la poche de fluide (1), **caractérisée en ce qu'**à l'intérieur du capuchon cassable (8) du dispositif de fermeture (4), un élément d'actionnement de soupape (9) est prévu, lequel, après la liaison de la tubulure de sortie (2) avec le dispositif de fermeture (4), commande par contrainte le corps de soupape (10) et ouvre ainsi le dispositif de soupape (3) de telle manière qu'il y a une liaison fluidique entre la poche de fluide (1) et une cavité (7) encapsulée par le capuchon cassable (8).

2. Poche de fluide selon la revendication 1,
**caractérisée en ce que** l'élément de base (5) du dispositif de fermeture (4) est réalisé en forme de cloche.

3. Poche de fluide selon l'une quelconque des revendications précédentes, **caractérisée en ce que** l'élément de base (5) est disposé par correspondance de matière sur la tubulure de sortie (2).

4. Poche de fluide selon l'une quelconque des revendications précédentes, **caractérisée en ce que** le capuchon cassable (8) est réalisé en forme de pot et l'élément d'actionnement de soupape (9) est prévu dans la cavité encapsulée en tant que saillie en forme de tige formée d'une seule pièce de matière avec le capuchon cassable (8), qui à l'état de montage laisse entre elle et une ouverture de soupape, une fente de flux d'évacuation et une ouverture de soupape dans la cavité encapsulée.

5. Poche de fluide selon l'une quelconque des revendications précédentes, **caractérisée en ce que** le dispositif de fermeture (4) est réalisé sous forme de fermeture inviolable.

6. Poche de fluide selon l'une quelconque des revendications précédentes, **caractérisée en ce que** le corps de soupape (10) est un poussoir de soupape en forme de douille, dont un côté frontal dirigé vers la poche de fluide (1) est fermé par une membrane, laquelle est soutenue par une broche centrale (13) sur la face intérieure de la poche pour guider un déplacement axial du corps de soupape pour une ouverture et une fermeture de la soupape (3).

7. Poche de fluide selon l'une quelconque des revendications précédentes, **caractérisée en ce qu'**elle peut être stérilisée à la vapeur chaude y compris l'espace encapsulé.

8. Poche de fluide selon l'une quelconque des revendications précédentes, **caractérisée en ce que** tous les composants de la poche de fluide (1), de la soupape (3) et du dispositif de fermeture (4) sont formés en matières plastiques thermoplastiques.

9. Poche de fluide médicale, en particulier poche de fluide de dialyse, laquelle présente au moins une tubulure de sortie (2), qui est fermée vers l'environnement extérieur avec un dispositif de soupape commutable (3) et est encapsulée au moyen d'un dispositif de fermeture (4) disposé de manière stérile et étanche sur la tubulure de sortie (2), qui présente un élément de base (5) agencé sur la tubulure de sortie (2) et un capuchon cassable (8),
dans laquelle l'élément de base (5) et le capuchon cassable (8) sont reliés l'un à l'autre d'un seul tenant par le biais d'une liaison (6) servant de section de rupture intentionnelle, et
dans laquelle le dispositif de soupape (3) s'ouvre après le retrait du capuchon cassable (8) en cas d'utilisation conforme à l'affectation de la poche de fluide (1) au moyen d'un système de raccordement, **caractérisée en ce qu'**un élément d'actionnement de soupape (9) est prévu à l'intérieur du capuchon cassable (8) du dispositif de fermeture (4), lequel après la liaison de la tubulure de sortie (2) avec le dispositif de fermeture (4) ouvre le dispositif de soupape (3) (au moins temporairement) de telle manière qu'il y a une liaison fluidique entre la poche de fluide (1) et une cavité (7) d'une zone de connexion (7a) pour le raccordement d'un système de raccordement à la poche de fluide (1).

10. Poche de fluide selon l'une quelconque des revendications précédentes, **caractérisée en ce que** l'élément d'actionnement de soupape (9) est une tige axialement en saillie, qui présente une section transversale cylindriquement massive, tubulaire, profilée, en particulier cruciforme, triangulaire ou en forme de Y.
